# EUROPEAN PATENT APPLICATION

(11) **EP 4 710 768 A1**
(43) Date of publication of application: **18.03.2026**
(21) Application number: 25167426.3
(22) Date of filing: 31.03.2025
(51) Int. Cl.: A01P 3/00

(54) **BACILLUS VELEZENSIS STRAIN VM02, A STIMULATING AGENT FOR THE GROWTH AND PRODUCTION OF HORTICULTURAL CROPS AND THE CONTROL OF PHYTOPATHOGENIC FUNGI**

(30) Priority: 16.09.2024 ES 202430729
(71) Applicant: Vellsam Materias Bioactivas, SL, 04200 Tabernas Almería (ES)
(72) Inventor: DE MONTIJO PRIETO, Soumi Manitou, 04200 Tabernas, Almería (ES); CASTRO MEDINA, David Jonathan, 04200 Tabernas, Almería (ES); PÉREZ MOLINA, Gema Maria, 04200 Tabernas, Almería (ES); PÉREZ RUEDA, Marina, 04200 Tabernas, Almería (ES)
(74) Representative: Anidjar Mogeda, Miriam

(57) **Abstract**

The present invention refers to the strain VM02 of the species *B*. *velezensis,* deposited in the Spanish Type Culture Collection under CECT number 30277, for application in horticultural crops as a biofertilizer and antagonist of phytopathogenic fungi. The strain VM02 has the ability to fix nitrogen, solubilize phosphates, produce ACC deaminase, and produce siderophores. The invention involves the evaluation of the strain's antifungal activity against pathogenic fungi of agricultural crops. The invention also includes the doses and manners of application of products containing the VM02 strain, which stimulate the plant and reproductive development of melon, watermelon, and pepper crops, and also increase the production and fruit quality. Likewise, the invention includes the dose and manner of application of products containing VM02 with the ability to inhibit the fungus *Podosphaera fusca* in zucchini crops.

## Description

### FIELD OF THE INVENTION

The present invention pertains to the agricultural sector as the field of application and refers to the strain VM02 of the species *Bacillus velezensis* (CECT 30277), with stimulating properties for plant growth and the control of pathogenic fungi in plants.

### BACKGROUND TO THE INVENTION

The use of microbial preparations as stimulating agents for plant growth and biological control of pests and diseases is one of the most promising alternatives for reducing the harmful effects of the indiscriminate use of chemicals in the agricultural sector.

Plant Growth-Promoting Rhizobacteria *(PGPR*) are a group of beneficial bacteria that live in the soil adjacent to plant roots. These bacteria have been extensively studied as potential substitutes for pesticides due to their ability to colonize plant roots and secrete a wide range of beneficial substances for plant growth, as well as antagonistic agents for the growth and invasion of pathogens [Beneduzi et al., 2012, Genet Mol Biol 35(4):1044-51]

Among the bacteria's main mechanisms of action associated with the promotion of plant growth are: the ability to fix atmospheric nitrogen; the solubilization of inorganic nutrients which limit plant growth speed, including the increase in phosphorus and available potassium; modulation of regulatory mechanisms in plants, such as the production of auxin, gibberellin, and cytokinin hormones, and the reduction of the plants' ethylene levels; and induction in the production of other compounds involved in plant development [Lugtenberg and Kamilova, 2009 Annu Rev Microbiol 63: 541-556].

Among the mechanisms of action of PGPR bacteria associated with pathogen antibiosis are: the ability to produce substances or metabolites such as siderophores, antibiotics, and surfactants that eliminate pathogenic fungi (fungicidal effect) or prevent the germination of their spores (fungistatic effect), the production of hydrolytic enzymes, such as glucanases and chitinases, and the modification of the pH of the rhizosphere via the production of acids, which also prevents the proliferation of pathogens. At the same time, these microorganisms can act as elicitors of jasmonic acid-dependent induced systemic resistance (ISR), thereby increasing resistance to pathogens [Delgado-Oramas et al., 2020, Rev. Protection Veg. 35(1)].

Given the metabolic variety of the species of the bacterial genus *Bacillus,* the number of strains and products based on this group of microorganisms for these purposes is increasing. Inoculation of plants with species of this genus results in an effective strategy to stimulate crop growth and production, while increasing resistance to pathogen infections.

Species of the genus *Bacillus* are ubiquitous in nature and are found in many types of soils. They have the potential to colonize the rhizosphere and penetrate the tissues of many plant species, promoting their growth and increasing production. These spore-forming microorganisms can act via the fixation of atmospheric nitrogen, the solubilization of phosphorus and potassium, the synthesis of phytohormones, and the production of secondary metabolites including antibiotics, lipopeptides, and siderophores. The actions of these mechanisms increase the plant's tolerance to abiotic stresses, such as drought, heat, and salinity, and to biotic stress caused by microorganisms, such as fungi, bacteria, viruses, and nematodes [Yi et al., 2022, Microorganisms 10(8):1682].

Phytopathogenic fungi are one of the main microbial groups that cause disease in plants, causing enormous losses in production. Many of these pathogens belong to the genera: *Botrytis, Pythium, Rhizoctonia, Alternaria, Fusarium, Phytophthora,* among many others, and can survive in the soil for years. Among the mechanisms of action of the species of the genus *Bacillus* that may explain the phenomenon of biological control of pathogens include: antibiosis, cross-protection, competition and induction of systemic resistance, among others [Ali et al., 2020, Microbiol. Res. 232: 126389].

There are several rhizobacteria-based products on the market with properties that promote plant growth and pathogen control, including various species of the genus *Bacillus* and Gram-negative bacteria belonging to the genera *Pseudomonas, Rhizobium,* and *Serratia.* Nonetheless, the use of new bacterial strains capable of improving crop production, with proven efficacy against phytopathogens and for specific crops, is one of the most urgent needs in the agronomy sector. The use of new, selectively targeted biofungicides can counter the economic losses caused by fungal diseases and constitutes an alternative to the use of chemical fertilizers to reduce harmful effects on the environment and, consequently, on human health.

### EXPLANATION OF THE INVENTION

The subject matter of this invention is the strain VM02 of the species *Bacillus velezensis,* as well as its mutants and metabolites. *Bacillus velezensis* strain VM02 was isolated from vermicompost by the company Vellsam Materias Bioactivas S.L. in July 2020 in Tabernas (Almeria, Spain). The isolation and selection of this strain was based on the rapid growth in minimal culture media and a wide pH range, as well as its ability to promote plant growth and inhibit phytopathogenic fungi. The identification of the species from the selected isolate was done by partial gene sequencing of the 16S ribosomal RNA in both directions. Analysis of the sequence of 1,348 base pairs resulted in 100% similarity with the *Bacillus velezensis* strain KKLW (GenBank CP054714.1). Additionally, its complete genomic DNA was sequenced at the Central Service for Experimental Research (SCSIE) at the University of Valencia (Valencia, Spain) using the *IlluminaMiSeq* platform, indicating that it contains 4,174,308 nucleotides, 4,136 proteins, and a guanine-cytokine (GC) content of 46.2%. It was identified under the code VM02 within the collection of microorganisms of Vellsam Materias Bioactivas S.L. and was subsequently deposited in the Spanish Type Culture Collection (CECT) under the number CECT 30277 on January 15, 2021.

The B. *velezensis* strain VM02 is a Gram-positive, catalase-positive bacillus, capable of growing at NaCl concentrations between 0.5 and 12 %, and a pH between 4 and 8.5. It grows at temperatures ranging from 10 to 40 °C, with the optimal temperature being 30 °C. It gives rise to white, smooth, circular colonies with entire margins and central oval spores.

The strain VM02of *B. velezensis* is capable of stimulating plant growth through various mechanisms. The ability to fix nitrogen was verified by cultivating the strain in plates with Burk culture medium, a medium with no nitrogen in its composition [Stella et al., 2010, J Trop Agric and Fd Sc 38(2): 211-219]. The growth of the strain VM02 in this medium demonstrated its ability to capture and use atmospheric nitrogen that can be used by the plant.

The ability of the strain *B. velezensis* VM02 to modulate concentrations of ethylene in the plant, produced in response to biotic and abiotic stress, was verified by growing the strain in minimal medium supplemented with 1-aminocyclopropane-1-carboxylic acid (ACC), a precursor to ethylene [Poonguzhali et al., 2006, Plant Soil 286: 167]. The use of this medium for the growth of the strain VM02 verifies the production of the hydrolytic enzyme 1-amino-cyclopropane-1-carboxylate deaminase (ACC-deaminase), which degrades ACC to ammonium and α-ketobutyrate, and thus reduces ethylene formation and its negative effects on plant growth and production [Mehak et al., 2023, Microbial Endophytes and Plant Growth, Academic Press: 151-166].

*B. velezensis* VM02 is capable of producing siderophores, iron chelating molecules that favor bioavailability in the rhizosphere. This PGPR property of the strain VM02 was verified by adding an overlay of chrome azurol S medium (CAS) [Louden et al., 2011, J Microbiol Biol Educ 12(1): 51-53] to the culture of the strain in nutrient agar. The presence of a translucent halo around the growth indicated production of siderophore molecules by the VM02 strain.

The ability of *B. velezensis* VM02 to solubilize phosphates was verified by growing the strain in NBRIP medium with tricalcium phosphate. The appearance of a translucent halo around the growth of the strain confirmed its ability to produce organic acids, which are useful for the conversion of insoluble phosphate forms into soluble forms that can be absorbed by plant roots. More specifically, the strain *B. velezensis* VM02 has the ability to solubilize and release 250 ppm of phosphate ion (PO4⁻) from tricalcium phosphate in Pikoskaya medium, which is equivalent to 5% of the insoluble phosphate present. This quantification was performed using the vanadate-molybdate method [Ou et al., 2022, Front Plant Sci. 13:880125].

The *B. velezensis* strain VM02 and its metabolites inhibit the growth of phytopathogenic fungi: *Alternaria alternata, Botrytis cinerea, Botrytis* sp., *Curvularia* sp., *Fusarium solani, Fusarium* sp., *Giberella zeae, Magnaporthe oryzae, Phytophthora cinnamomi, Phyllosticta cucurbitacearum, Verticillium dahliae, Monilinia fructigena, Sclerotinia sclerotiorum, Penicillium digitatum,* and *Fusarium circinatum.* This antifungal property of the strain and its metabolites was evaluated via *in vitro* antagonism tests in Petri dishes with potato-dextrose agar (PDA) and in liquid medium with a potato-dextrose broth, respectively.

The *B. velezensis* strain VM02 is distinctive for its easy production and scaling in liquid medium, as well as the rapid production of resistance spores, which allows it to survive for long periods of time and remain viable in formulated products incorporating it.

The present invention refers to the *B. velezensis* strain VM02 and its metabolites, with biostimulant and biofungicidal ability, to be applied in agronomy crops.

In a preferential embodiment of the invention, products prepared with the *B. velezensis* strain VM02 contain a concentration between 10^6 and 10^9 CFU/ml (colony-forming units per ml), stabilized in organic matter to be used in roots with biostimulant and biofungicidal activity.

In a preferential embodiment of the invention, products prepared with the *B. velezensis* strain VM02 and its metabolites contain a concentration between 10^8 and 10^9 CFU/ml (colony-forming units per ml) for foliar use with biofungicidal activity.

The present invention also refers to the bacterium *B. velezensis* VM02 as a biostimulant agent with the ability to increase the production of horticultural crops and fruit quality.

More specifically, it refers to the strain *B. velezensis* VM02, or a product containing it, with the ability to promote vegetative and reproductive development, as well as production and production quality in melon crops.

More specifically, it also refers to the strain *B. velezensis* VM02, or a product containing it, with the ability to increase crop yields and the parameters of fruit quality in watermelon crops.

More specifically, it also refers to the strain *B. velezensis* VM02, or a product containing it, with the ability to promote plant growth, nutritional status, and production in pepper crops.

The strain B. *velezensis* VM02, or a product containing it, is additionally the subject matter of the present invention as an agent of control for the fungi causing phytopathologies in agronomy crops.

In a particular embodiment, it refers to the strain *B. velezensis* VM02 with the ability to control powdery mildew *(Podosphaera fusca)* in cucurbits, specifically, in zucchini crops.

### BRIEF DESCRIPTION OF THE DRAWINGS

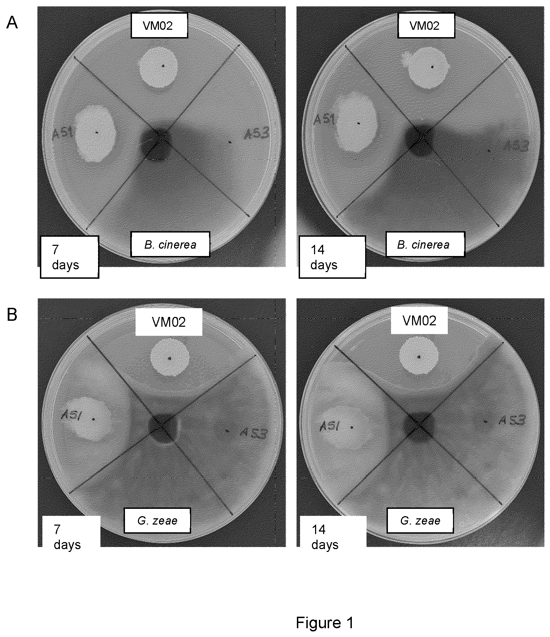

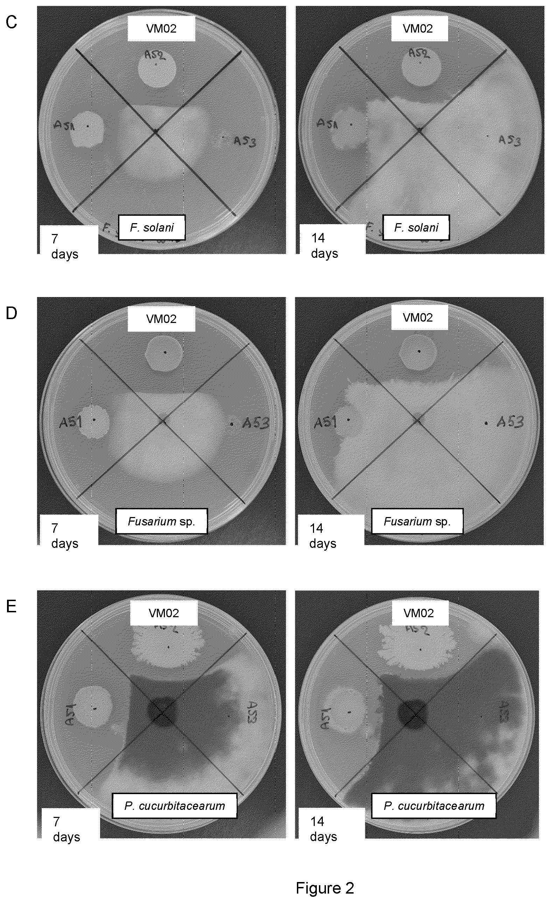

**Figure 1****: Inhibition of phytopathogenic fungi by the bacterium VM02.**
The inhibition of the growth of some phytopathogenic fungi by the bacterium VM02 and its metabolites after 7 and 14 days of incubation is shown. The fungi shown in the photos correspond to: *Botrytis cinerea (A), Giberella zeae (B), Fusarium solani (C), Fusarium* sp. (D), and *Phyllosticta cucurbitacearum (E).*

### PREFERENTIAL EMBODIMENT OF THE INVENTION

The invention is described below with examples of illustrative embodiments, but not limited to these.

### Example 1: Evaluation of the antifungal activity of the B. velezensis strain VM02 against phytopathogenic fungi

The antifungal activity of the strain *B. velezensis* VM02 was evaluated against the fungi: *Alternaria alternata, Botrytis cinerea, Botrytis* sp., *Curvularia* sp., *Fusarium solani, Fusarium* sp., *Giberella zeae, Magnaporthe oryzae, Phytophthora cinnamomi, Phyllosticta cucurbitacearum, Verticillium dahliae, Monilinia fructigena, Sclerotinia sclerotiorum, Penicillium digitatum* and *Fusarium circinatum.* A 4 mm piece of fungus mycelium was cultivated in the center of a petri dish with dextrose potato agar (PDA), and 2.5 cm apart, 10 µl of a culture of the VM02 strain was inoculated for 18 hours of growth. The section of the plate without bacterial inoculum was used as a positive control of the fungal growth. After incubation of the plates between 22 and 25 °C, the area of growth of each fungal mycelium was measured, and the percentage of inhibition compared to the growth in the control area was calculated. As can be seen in Table 1, the VM02 strain inhibited the growth of the evaluated fungi between 57.7 and 96.7% after 7 days of incubation, and between 49.1 and 96.5% after 14 days of incubation.

**Table 1. In vitro inhibition of phytopathogens by Bacillus velezensis strain VM02**

| | **Percentage of fungal inhibition (%)** | |
|---|---|---|
| | **7 days** | **14 days** |
| *Alternaria alternata* CECT 2662 | 88.3 | 86.5 |
| *Gibberella zeae* CECT 2150 | 79.0 | 76.2 |
| *Botrytis cinerea* CECT 2100 | 96.7 | 96.5 |
| *Verticillium dahliae* WT | 91.0 | 95.5 |
| *Fusarium* sp. WT | 77.7 | 85.0 |
| *Botrytis* sp. WT | 81.8 | 91.1 |
| *Curvularia* sp. WT | 80.9 | 86.4 |
| *Fusarium solani* WT | 81.6 | 91.1 |
| *Phyllosticta cucurbitacearum* WT | 87.6 | 85.0 |
| *Fusarium circinatum* WT | 57.7 | 49.1 |
| *Sclerotinia sclerotiorum* | 91.1 | 94.7 |
| *Monilinia fructigena* | 86.4 | 87.8 |
| *Penicillium digitatum* | 81.0 | 81.9 |
| *Magnaporthe orvzae* | 87.1 | 85.3 |

| | | |
|---|---|---|
| CECT: Spanish Type Culture Collection; WT: wild type | | |

The antifungal activity of the metabolites produced by the B. *velezensis* strain VM02 was carried out in dextrose potato broth (PDB) by combining 300 µl of supernatant of the VM02strain, previously centrifuged and filtered with a 0.22 µm membrane, with 900 µl of a suspension of the fungus in fresh medium, standardized to 10^6 propagules/ml. After 14 days of incubation between 20 and 25 °C, the supernatants of the strain VM02 inhibited the growth of the fungi *Magnaporthe oryzae, Sclerotinia sclerotiorum, Monilinia fructigena,* and *Penicillium digitatum.*

### Example 2: Evaluation of the ability of the Bacillus velezensis strain VM02 to promote plant and reproductive development, as well as production and production quality in melon crops

The stimulating activity of the *B. velezensis* strain VM02 on a melon crop was evaluated via root application of a product with the strain, performed on an experimental farm. Water was used in the control group as a negative control without bacterial inoculant. The product containing the VM02 strain and the control were both applied via irrigation. The treatments are described below in sections I and II.
I. Treatment, negative control (irrigation water) - without inoculant
II. Treatment with the product containing the VM02 strain, applied to the roots in a range of 1-10 L/ha.

Two samplings were conducted during the crop cycle at 28 and 54 days after transplantation to monitor the vegetative and reproductive development of the treated plants. The impact of the strain on the growth of the crop in the early phenological stages, considered to be the first 60 days after transplantation, was evaluated. Additionally, values related to the production and production quality of the plant system under analysis were evaluated. The nutritional status of the crop and a soil analysis were also performed to complete the evaluation of this trial. Tables 2, 3, and 4 show the results of the trial.

**Table 2. Physiological parameters of a melon crop after the application of VM02**

| | **Treatment** | **Height (cm)** | **Thickness (mm)** | **Leaves (units·plant⁻¹)** | **SPAD** |
|---|---|---|---|---|---|
| 28 DDT | Control | 84.57 ± 9.42 a | 7.10 ± 0.92 a | 25.97 ± 3.45 a | 25.78 ± 1.38 a |
| | Product VM02 | 96.67 ± 6.22 b | 8.14 ± 1.02 b | 24.50 ± 3.35 a | 26.52 ± 1.78 b |
| 54 DDT | Control | 183.63 ± 11.53 a | 7.10 ± 0.92 a | 19.93 ± 2.30 a | 43.67 ± 2.61 a |
| | Product VM02 | 203.13 ± 23.77 b | 8.14 ± 1.02 b | 30.03 ± 5.78 b | 45.93 ± 2.32 b |

| | | | | | |
|---|---|---|---|---|---|
| *The distinct letters a-b denote the existence of significant differences according to LSD Fisher (p<0.05).* *DDT: days after transplantation. NS: Not significant* | | | | | |

**Table 3. Production parameters of a melon crop after the application of VM02**

| **Treatment** | **Yield (kg/m²)** | **Production increase (%)** |
|---|---|---|
| Control | 2.85 | |
| Product with VM02 | 3.99 | 39 |

**Table 4. Production quality of a melon crop after the application of VM02**

| **Treatment** | **Weight (g)** |
|---|---|
| Control | 3.27 ± 0.41 a |
| Product with VM02 | 3.85 ± 0.64 b |

| | |
|---|---|
| *The distinct letters a-b denote the existence of significant differences according to LSD Fisher (p<0.05).* | |

The product with the VM02 strain stimulated plant growth at all stages during the crop cycle (Table 2). Specifically, plant height and stem thickness were higher than the values of plants without the product in the two samplings that were conducted. Additionally, the number of leaves in the second sampling increased as a result of a cumulative effect after three applications of the product with strain VM02. In addition, the value of the SPAD index also increased significantly throughout the trial. Analysis of the production and production quality revealed that the application of the product with inoculum of the bacterial strain, subject matter of the invention, resulted in the production of a greater number of fruits per plant with higher weights and an increase in productive yield (Tables 3 and 4).

### Example 3: Evaluation of the ability of the B. velezensis strain VM02 to increase crop yield and parameters of fruit quality in watermelon crops

The stimulating activity of the *B. velezensis* strain VM02 on a watermelon crop was evaluated via root application of a product containing the strain, performed on an experimental farm. Water was used in the control group as a negative control without microbial inoculant. The product containing the VM02 strain and the control were both applied via irrigation. The treatments are described below in sections I and II. The efficacy of the treatments on crop yield and fruit quality was evaluated (Tables 5 and 6).
I. Treatment, negative control (irrigation water) - without inoculant
II. Treatment with the product containing the VM02 strain, via root application at a ratio of 2 L/ha.

**Table 5. Production parameters of a watermelon crop after the application of VM02**

| **Treatment** | **Yield (kg/m²)** | **Production increase (%)** |
|---|---|---|
| Control | 5.42 | |
| Product with VM02 | 6.30 | 16.24 |

**Table 6. Production quality in a watermelon crop after the application of VM02**

| **Treatment** | **Weight (kg)** | **° Brix** | **Cell wall (cm)** |
|---|---|---|---|
| Control | 4.35 ± 0.58 a | 9.20 ± 0.83 a | 0.94 ± 0.05 a |
| Product with VM02 | 5.00 ± 0.40 b | 11.85 ± 1.02 b | 1.14 ± 0.10 a |

| | | | |
|---|---|---|---|
| *The distinct letters a-b denote the existence of significant differences according to LSD Fisher (p<0.05).* | | | |

As can be seen in Table 5, the application of the product with the B. *velezensis* strain VM02 during the crop cycle increased watermelon production. Likewise, production quality increased given that a higher weight and average size of the fruit were induced, as well as increased wall thickness in the fruits of the experimental group that was subjected to the bacterial treatment (Table 6).

### Example 4: Evaluation of the ability of the B. velezensis strain VM02 to promote plant growth, nutritional status, and production in a pepper crop

The stimulating activity of the B. *velezensis* strain VM02 on a pepper crop was evaluated via root application of a product with the strain, performed on an experimental farm. The product with the VM02 strain, as well as its controls, were applied via irrigation. The treatments applied are described below in sections I, II, and III.
I. Control treatment (irrigation water) - without bacterial inoculant.
II. Treatment with the product containing the VM02 strain, via root application in a range of 1-10 L/ha.

Parameters related to the physiological and reproductive aspects of the crop were evaluated [Acevedo and Pire, 2004, Interciencia 29 (5), 274-279], as well as production and quality. In addition, analyses of soil fertility and nutrition in leaf tissues were conducted to determine the impact of the bacterial strain on the availability of nutrients in the soil and their absorption by the plant. Tables 7 - 10 show the statistical study of the parameters under analysis. Three harvests (C1, C2 and C3) were collected during the crop cycle to evaluate fruit quality.

**Table 7. Physiological parameters of a pepper crop after the application of VM02**

| | **Treatment** | **Height (cm)** | **Thickness (mm)** | **Leaves (units·plant⁻¹)** | **SPAD** |
|---|---|---|---|---|---|
| 29 DDT | Control | 52.90 ± 2.77 a | 6.80 ± 0.70 a | 31.73 ± 2.44 a | 42.62 ± 1.21 a |
| | Product VM02 | 58.07 ± 3.35 b | 7.20 ± 0.70 a | 34.63 ± 2.55 b | 44.19 ± 1.45 b |
| 43 DDT | Control | 77.53 ± 3.35 a | 10.30 ± 0.60 a | 42.97 ± 2.55 a | 44.31 ± 2.55 a |
| | Product VM02 | 82.00 ± 4.04 b | 10.90 ± 0.80 a | 46.63 ± 2.12 b | 46.06 ± 1.62 b |

| | | | | | |
|---|---|---|---|---|---|
| *The distinct letters a-b denote the existence of significant differences according to LSD Fisher (p<0.05).* *DDT: days after transplantation.* | | | | | |

**Table 8. Nutritional content of leaf tissue of a pepper crop after the application of VM02**

| | **Flowerin g** | | **Fruiting** | | **Units** |
|---|---|---|---|---|---|
| | **Contro** l | **Product VM02** | **Control** | **Product VM02** | |
| **Total N** | 4.76 | **5.01** | 4.80 | **5.39** | % |
| **P** | 0.24 | 0.24 | 0.27 | **0.29** | % |
| **K** | 6.36 | **6.56** | 3.18 | **3.37** | % |
| **Mg** | 0.51 | **0.53** | 0.28 | **0.36** | % |
| **Ca** | 1.30 | **1.45** | 1.70 | **1.94** | % |
| **Na** | 0.01 | **0.02** | 0.01 | 0.01 | % |
| **Zn** | 51.50 | **55.50** | 40 | 36.00 | mg/kg |
| **Fe** | 60.00 | **63.00** | 49.50 | **51.90** | mg/kg |
| **Mn** | 76.50 | 69.50 | 53.50 | **57.00** | mg/kg |
| **Cu** | 19.00 | 16.50 | 10.00 | 9.00 | mg/kg |
| **B** | 32.50 | 28.50 | 28.00 | 22.5 | mg/kg |

| | | | | | |
|---|---|---|---|---|---|
| *Results expressed for dry matter.* | | | | | |

**Table 9. Production parameters of a pepper crop after the application of VM02**

| **Treatment** | **Yield (kg/m²)** | **Production increase (%)** |
|---|---|---|
| Control | 6.21 | |
| Product VM02 | 7.47 | 20 |

**Table 10. Production quality of a pepper crop after the application of VM02**

| | **Treatment** | **Weight (g)** | **Length (cm)** | **Diameter (cm)** | **Cell wall (mm)** |
|---|---|---|---|---|---|
| C1 | Control | 180.70 ± 12.81 a | 8.09 ± 0.64 a | 8.63 ± 0.43 A | 7.10 ± 0.77 A |
| | Product VM02 | 194.50 ± 14.63 b | 8.30 ± 0.69 b | 8.84 ± 0.40 A | 8.46 ± 0.82 b |
| C2 | Control | 221.53 ± 20.78 a | 8.95 ± 0.98 a | 9.19 ± 0.48 A | 7.23 ± 0.50 A |
| | Product VM02 | 256.43 ± 21.54 b | 8.90 ± 0.83 a | 8.85 ± 0.44 A | 7.92 ± 0.43 b |
| C3 | Control | 282.03 ± 20.62 a | 10.01 ± 0.78 a | 8.43 ± 0.62 A | 7.38 ± 0.57 A |
| | Product VM02 | 318.30 ± 15.05 b | 10.68 ± 0.86 a | 9.69 ± 0.81 b | 8.21 ± 0.86 b |

| | | | | | |
|---|---|---|---|---|---|
| *The distinct letters a-b denote the existence of significant differences according to LSD Fisher (p<0.05).* *C1: harvest 1; C2: harvest 2; C3*: *harvest 3.* | | | | | |

As demonstrated in Table 7, the group treated with the product of the VM02 strain had greater plant height throughout the trial at the physiological level, both at 29 and 43 days. The quantification of the foliage of the group treated with the VM02 strain was statistically superior in both samplings compared to the control. Likewise, the values of the SPAD index or chlorophyll content remained higher in the group treated with the VM02 strain.

The nutritional analysis of the leaf tissue was performed at two key stages of the crop, flowering and fruiting (Table 8). The contents of total nitrogen (total N), potassium (K), magnesium (Mg), and calcium (Ca), expressed in percentages, were higher in the plants treated with the product containing VM02 in both stages of the crop. The phosphorus (P) content was increased in the fruiting stage compared to the control group. The values of sodium (Na) during the flowering stage were higher than the control group, as was zinc (Zn).

The production and yield of the crop increased by 20% with the application of the VM02 product (Table 9). Likewise, fruit quality with regard to weight and cell walls was improved with the application of the product containing the VM02 strain (Table 10) in all the harvests evaluated (C1, C2, and C3). Meanwhile, the length of the fruit increased only significantly in the first harvest, and the diameter of the fruit (size) in the third harvest.

### Example 5: Evaluation of the effect of the application of the B. velezensis strain VM02 on the control of powdery mildew (Podosphaera fusca)

The biofungicidal activity of the *B. velezensis* strain VM02 was evaluated via foliar application of a product on a zucchini crop infected with the fungus *Podosphaera fusca,* performed on an experimental farm. Sections I, II, and III describe the treatments applied:
I. Treatment, negative control (irrigation water) - without inoculant
II. Treatment with an organic fungicide with a vegetable oil base
III. Treatment with a product containing the VM02 strain, via foliar application in a range between 2 and 10 % w/v.

For this evaluation, the plants affected by the fungus in each experimental group were counted and the leaf area covered with the pathogen was estimated using a severity scale in order to calculate the disease index [McKinney, 1923, J. Agric. Res. 26, 195]. These two parameters were measured before and after the treatments to determine efficacy. The evolution of the disease was monitored throughout the efficacy trial every 7 days after the first application and before the next treatment. The results are shown below in Tables 11-12.

**Table 11. Severity of P. fusca infection in a zucchini crop after the application of VM02**

| | **Treatment** | **McKinney Index** |
|---|---|---|
| 7 DDA | Control | 33.25 ± 11.02 ns |
| | Organic Funqicide | 30.48 ± 10.40 ns |
| | Product VM02 | 32.33 ± 15.47 ns |
| 15 DDA | Control | 50.60 ± 12.34 b |
| | Organic Funqicide | 47.47 ± 12.65 b |
| | Product VM02 | 29.02 ± 12.69 a |
| 25 DDA | Control | 57.45 ± 11.35 c |
| | Organic Funqicide | 46.07 ± 10.55 b |
| | Product VM02 | 26.40 ± 13.51 a |
| 33 DDA | Control | 69.13 ± 14.26 c |
| | Organic Funqicide | 52.93 ± 11.76 b |
| | Product VM02 | 22.67 ± 9.89 a |

| | | |
|---|---|---|
| *The distinct letters a-c denote the existence of significant differences according to LSD Fisher (p<0.05). DDA: days after the first application* | | |

**Table 12. Biological efficacy of the treatment with VM02 against P. fusca infection in a zucchini crop**

| | **Treatment** | **Biological efficacy** |
|---|---|---|
| 7 DDT | Organic Funqicide | 8.33 a |
| | Product VM02 | 2.77 a |
| 15 DDT | Organic Funqicide | 6.19 a |
| | Product VM02 | 42.65 b |
| 25 DDT | Organic Fungicide | 19.81 a |
| | Product VM02 | 54.05 b |
| 33 DDT | Organic Funqicide | 23.43 a |
| | Product VM02 | 67.21 b |

| | | |
|---|---|---|
| *The distinct letters a-b denote the existence of significant differences according to LSD Fisher (p<0.05).* | | |

The application of the product containing the VM02 strain revealed lower severity according to the McKinney scale (Table 11). It also showed a high biological efficacy in inhibiting the development of the fungus in zucchini (Table 12). It is an alternative for the biocontrol of the agent P. *fusca* compared to other commercial biofungicides with a plant oil extract base, which have a lower efficacy as previously demonstrated. Therefore, it can be concluded that the systematic application of the product containing the *Bacillus velezensis* strain VM02 induced the plant's resistance and also enhanced its ability to stop the disease and prevent the spread of the phytopathogen.

## Claims

1. Strain VM02 of the species *Bacillus velezensis,* deposited in the Spanish Type Culture Collection (CECT) under number 30277.

2. A culture of *Bacillus velezensis* strain VM02 (CECT 30277) and its mutants, with the ability to stimulate growth and production in crops through the fixation of atmospheric nitrogen, solubilization of phosphorus, production of ACC-deaminase enzymes, production of siderophores, and production of indole acetic acid.

3. A product that stimulates the growth of horticultural crops and the biological control of plant pathogenic fungi, **characterized by** the fact that it contains viable cells of the strain VM02 of the species *Bacillus velezensis* (CECT 30277).

4. The product of claim 3 containing *Bacillus velezensis* strain VM02 (CECT 30277) with a concentration of viable cells between 10^5 and 10^10 CFU/ml.

5. The product of claim 3 containing *Bacillus velezensis* strain VM02 (CECT 30277) with a concentration of viable cells between 10^5 and 10^10 CFU/ml, additionally containing secondary metabolites of the strain VM02.

6. The product in accordance with claims 4 or 5, which maintains the viability of the strain VM02 for at least one year when stored between 0 and 40 °C.

7. The product in accordance with claims 4 or 5 with the ability to stimulate growth in horticultural crops when applied via the roots.

8. The product according to claim 7 with the ability to promote plant and reproductive development, as well as production and production quality in melon crops.

9. The product according to claim 7 with the ability to increase crop yield and the parameters of fruit quality in watermelon crops.

10. The product according to claim 7 with the ability to promote plant growth, nutritional status, and production in pepper crops.

11. The product of claim 5 which stimulates plant and reproductive development of crops and increases the production and fruit quality.

12. The product of claim 5 that inhibits the growth of pathogenic fungi in crops.

13. The product in accordance with claim 5, with fungicidal activity against the fungus *Podosphaera fusca* in zucchini crops when applied to the foliage.

14. The product in accordance with claim 2 or 3 in powder form with a concentration of viable cells between 10^8 and 10^12 CFU/g, via foliar and root application.
